# EUROPEAN PATENT SPECIFICATION

(11) **EP 4 353 287 B1**
(45) Date of publication and mention of the grant of the patent: **17.12.2025**
(21) Application number: 23201907.5
(22) Date of filing: 05.10.2023
(51) Int. Cl.: A61M 16/00, A61M 16/20, A63B 23/18

(54) **IMPROVED RESPIRATORY PHYSIOTHERAPY DEVICE**
VERBESSERTE ATEMPHYSIOLOGISCHE THERAPIEVORRICHTUNG
DISPOSITIF DE PHYSIOTHÉRAPIE RESPIRATOIRE AMÉLIORÉ

(30) Priority: 10.10.2022 IT 202200004083 U
(43) Date of publication of application: 17.04.2024
(73) Proprietor: Flaem Nuova S.p.A., 25015 Desenzano del Garda (BS) (IT)
(72) Inventor: ABATE, Riccardo, 25015 Desenzano del Garda (BS) (IT); ONGARETTI, Luca, 25060 Cellatica (BS) (IT); STEFANNI, Stefano, 25015 Desenzano del Garda (BS) (IT)
(74) Representative: Botti & Ferrari S.p.A.

(56) References cited:
- US-A- 5 193 529
- US-A1- 2010 101 573
- US-A1- 2012 097 164
- US-A1- 2014 150 801

## Description

### Field of application

In its more general aspect the present invention relates to a combined respiratory physiotherapy device for performing a variety of therapies.

The present invention finds its application particularly, but not exclusively, in the field of respiratory physiotherapy based on positive expiratory pressure.

### Prior art

In the present field it is known that respiratory physiotherapy aims at helping and rehabilitating patients suffering from acute and chronic respiratory diseases, increasing lung capacity, or helping to clear the airways of the secretions produced.

One of the most frequently prescribed airway clearance therapy (ACT) options for patients with chronic lung disease or reduced lung volumes are the Positive Expiratory Pressure therapy devices, or PEP ("Positive Expiratory Pressure") devices.

During the therapy with said devices, the patient exhales against a fixed orifice resistor, generating pressure during exhalation, which usually, according to various sources, range from 0.98 to 1.96 kPa.

The PEP therapy does not require an external pressurized gas source, but exclusively requires the patient's expiratory effort.

The therapy, which is administered by breathing through said devices, helps both moving the secretions, or mucus, present on the walls of the lungs and from the airways, and keeping the airways open for longer.

Increasing geriatric population along with higher incidence of chronic conditions drives the growth of the PEP device market.

For instance, according to a study carried out by the Centers for Disease Control and Prevention (CDC), in 2018, approximately 9 million adults have been diagnosed with chronic bronchitis which ultimately leads to higher requirements for PEP devices, fueling the market.

The increase in pressure caused by the passage of exhaled air through a calibrated orifice is transmitted to the airways, creating back pressure during exhalation, and generating passages, or "stents", thus preventing premature closure of the airways and reducing the gas trapping.

The PEP therapy promotes the collateral ventilation, allowing pressure to accumulate in the distal position with respect to the obstruction.

Specifically, the collateral ventilation is a phenomenon that occurs in the human lungs where the alveolar structures are ventilated through channels that bypass the normal airways, preserves the airways from collapse and prolongs the expiratory flow.

The PEP therapies currently adopted may be divided into two macro categories: the classic PEP and OPEP ("Oscillatory Positive Expiratory Pressure").

The devices adapted to carry out the classic PEP therapy were introduced at the end of the 1970s and aim at increasing the functional residual capacity (FRC), i.e. the volume of air present in the lung at the end of a normal exhalation, and the tidal volume (VT), i.e. the quantity of air that is mobilized with each non-forced breath.

Moreover, said devices increase the resistance to the expiratory air flow to facilitate the removal of secretions adhered to the path of the airways, preventing the closure of the airways themselves and increasing collateral ventilation.

Examples of this kind of devices are known with their brands: TheraPEP^{®}, Resistex PEP Mask and Pari PEP S System.

Conversely, the devices adapted to perform the OPEP therapy combine high-frequency airflow oscillations with positive exhalation.

Document US 2014/150801 A1 discloses an airway pressure control device with an oscillating pressure valve to control expiratory air flow. The device comprises a plug in an expiratory air passageway which is held against the passageway by an adjustable biasing spring. The spring is shaped or positioned or otherwise adapted to cause the forces acting on the stopper to be unbalanced as air flows around the stopper, causing the stopper to "flutter" and the pressure drop across the stopper to oscillate. Depending on the air pressure pushing against the plug bottom, the air flow around the plug is smooth and laminar or the plug flutters in the passageway, providing an oscillating pressure drop across the plug.

Therefore, they exploit resistance to make exhalation more difficult, like the devices that perform the classic PEP therapy, but further create vibrations when a patient exhales.

These vibrations move mucus from the surface of the airways.

After blowing several times through the device, the patient will thus be encouraged to expectorate to eliminate mucus from the lungs. Examples of this kind of devices are known with their brands: Flutter^{®}, Acapella^{®}, AerobikA^{®} and RC-Cornet^{®}.

As it may be inferred, the two kinds of devices comprise different features, which are targeted and specific for the chosen therapy on a case-by-case basis.

Should it be necessary, due to a change in therapy, to switch from the use of a PEP device to an OPEP device or vice versa, or should the same patient need different devices over time, it is therefore obvious that the expenses incurred will double, as well as the difficulty of managing two different devices.

For this reason, an object of the present invention is to solve the prior art drawbacks.

A further object is to provide a simple and practical solution even for an inexperienced user.

Still a further object of the invention is to provide a solution that allows an optimal operation in any chosen operating mode.

Another object is to provide a solution that may also be coupled to additional devices and scalable for adding more functionality.

Still a further object of the invention is to provide a device that does not need long cleaning times and minimizes the maintenance required. Finally, an object is to provide a solution that is structurally optimized even in terms of production and related costs.

### Summary

The solution idea underlying the present invention is to provide a device able to combine the features of the PEP devices and of the OPEP devices and to allow a user to alternatively carry out one or the other therapy.

Based on this solution idea the above objects are achieved by a respiratory physiotherapy device comprising a main body, a valve member adapted to adjust an inlet and outlet air flow from the main body allowing air to enter the device and hermetically blocks the outletting air, fluid connection means with the respiratory tract of a patient, said fluid connection means being connected to the main body and a convertible Positive Expiratory Pressure module comprising oscillating means.

The module is connected to the main body and is switchable between a first position in which the oscillating means are blocked in the module and a second position in which the oscillating means are movable in the module.

The invention is defined in the appended independent claim 1. Preferred embodiments are matter of the dependent claims.

Advantageously, therefore the present solution allows switching between the therapies carried out by activating the action of the oscillating means provided in the device or not.

Preferably, the above module comprises a first element coupled to the main body, the first element comprising a housing seat for said oscillating means, and a second element coupled to the first element and adapted to constrain and release the oscillating means from the housing seat. Advantageously, said solution ensures that the user decides with an intuitive action whether to exploit the vibration of the oscillating means or not, depending on the chosen therapy.

Still preferably, the second element of the above module comprises at least one cap portion adapted to be on top of the oscillating means on the housing seat of the first element.

Advantageously, this allows blocking or releasing the oscillating means by simply pressing them against the housing seat obtained in the first element.

More preferably, the oscillating means comprise a spherical body, preferably metallic.

Advantageously, said solution is optimal in terms of operation of the OPEP therapy, as well as in terms of carrying out the switching between therapies in the device.

The device according to the invention further comprises a setting ring nut configured to be couple to the module, the ring nut comprising at least one calibrated hole.

Advantageously, the present solution allows the user to simply rotate the ring to align the calibrated hole in order to obtain the correct desired resistance depending on the intensity of the PEP therapy prescribed. Preferably, the device according to the present invention further comprises an inlet plug coupled to the main body at the valve member. Advantageously, the present solution may also provide an inlet plug comprising the valve member so as to optimize the air inlet into the device and the hermetic block of the outlet air, in order to be able to put the device under perfect pressure during the delivery of the PEP or OPEP therapies.

Still preferably, the device according to the present invention further comprises an outlet plug coupled to the main body at the fluid connection means, preferably made up of a mouthpiece.

Advantageously, the present solution may also provide an outlet plug acting as adapter, made to couple different fluid connection means.

Still preferably, the device according to the present invention further comprises at least one T connecting element coupled between said main body and said fluid connection means, said T connecting element being adapted to be connected to a gauge.

Advantageously, the present solution allows the user, or preferably a third party such as a physiotherapist, to set the device according to the most suitable characteristics for the therapy necessary for the patient.

More preferably, the main body of the device according to the invention further comprises a containment housing for the module.

Advantageously, the present solution ensures ease of assembly and disassembly of the device, as well as a compact construction thereof.

Preferably, the device according to the present invention further comprises at least one grip knob adapted to be coupled to the main body.

Advantageously, the present solution allows a practical use of the device during therapy.

Further features and advantages of the improved respiratory physiotherapy device according to the present invention will become clearer from the following description of a preferred embodiment given by way of non-limiting example with reference to the enclosed figures.

### Brief description of the drawings

Figure 1 shows a perspective view of an improved respiratory physiotherapy device according to the present invention;
Figure 2 shows another perspective view of the improved respiratory physiotherapy device of Figure 1;
Figure 3 shows another perspective view of the improved respiratory physiotherapy device of Figure 1 coupled to a nebulization cup;
Figure 4 shows another perspective view of the improved respiratory physiotherapy device of Figure 1 coupled to a T connecting element;
Figure 5 shows an exploded view of the improved respiratory physiotherapy device of Figure 1 coupled to the nebulization cup;
Figure 6 shows a side view of the improved respiratory physiotherapy device of Figure 1;
Figures 7A and 7B show details of two operating conditions of the improved respiratory physiotherapy device of Figure 1;
Figures 8A and 8B show two sectional views of the improved respiratory physiotherapy device of Figure 1 in the operating conditions shown in Figures 7A and 7B;
Figure 9 shows a sectional view of the improved respiratory physiotherapy device of Figure 1 in a specific use condition.

In the different figures, similar elements will be identified by the same reference numbers.

### Detailed description

With reference to the enclosed figures, reference number 1 indicates an improved respiratory physiotherapy device according to the present invention, hereinafter called device 1, for the sake of brevity simply.

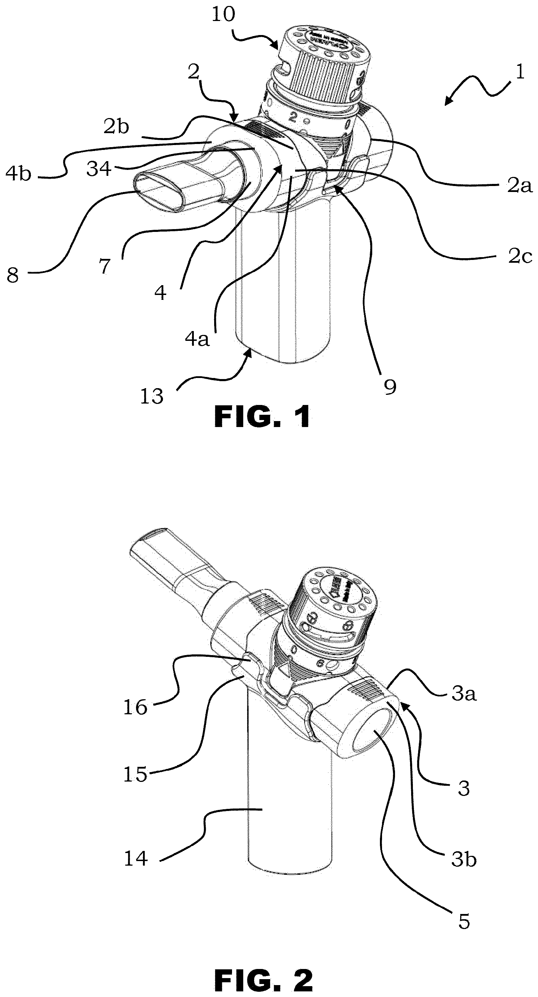

Looking specifically at Figures 1 to 4, the device 1 comprises a substantially tubular main body 2.

In the present embodiment the main body 2 is shaped like a hollow ellipsoidal prism.

Nothing prevents from providing different conformations, for instance with circular or polygonal section or others, all of the present variants being included in the scope of protection defined in the enclosed claims.

At the two ends 2a and 2b of the main body 2 an inlet plug 3 and an outlet plug 4, removably shape-coupled to the main body 2, are respectively provided.

The inlet plug 3 has a U cross section conformation, with a side wall 3a adapted to be coupled as extension of the main body 2 and a first bottom wall 3b.

At the bottom wall 3b of the inlet plug 3 an opening 5 is provided, which, in the present embodiment, is circular and coaxial with the main body 2.

A unidirectional valve member 6 is provided at the opening 5, said valve member 6 allowing air to enter the device 1 and hermetically blocks the outletting air.

The outlet plug 4, in the present embodiment, has a U cross section conformation, with a side wall 4a adapted to be coupled as extension of the main body 2 and a second bottom wall 4b.

Even in this case, at the bottom wall 4b an opening 34 is provided, which, in the present exemplifying embodiment, is circular and coaxial, and from which a hollow cylindrical portion 7 projecting from the bottom wall 4b departs.

Said hollow cylindrical portion 7 is adapted to couple fluid connection means with the airways of a patient, in the present exemplifying embodiment represented by a mouthpiece 8.

Nothing prevents from adopting different fluid connection means with the airways of a patient.

Furthermore, it is possible to provide for different outlet plugs 4, so that different hollow cylindrical portions 7 act as adapters to couple different fluid connection means.

At a substantially medial section of the main body 2, on a side wall 2c, a containment housing 9 is obtained.

Said containment housing 9 is adapted to house in shape-coupling a Positive Expiratory Pressure module, or PEP module, 10, that is substantially transversal with respect to the longitudinal development of the main body 2 between the two ends 2a and 2b.

The PEP module 10 comprises a first element 11, or PEP element, fitted in the containment housing 9.

The PEP module 10, at an end opposite the main body 2, also comprises a second element 12, or OPEP element, which, in the present exemplifying embodiment, is cap-shaped and adapted to be on top of the first element 11.

Nothing prevents from providing a different OPEP element 12, for instance shaped so as to always have a cap-shaped portion but having a wholly different conformation.

Furthermore, generally but not exclusively in a position opposite the PEP module 10, a knob 13 for a more comfortable grip of the device 1 is provided.

In the present exemplifying embodiment, the knob 13 comprises a cylindrical portion 14 and a U seat 15 that is substantially transversal to the cylindrical portion 14 and tilted toward the inlet plug 3, with four hooking edges 16, the main body 2 being adapted to be removably snap inserted into the seat 15.

Nothing prevents from adopting different knobs with different hooking systems or even not from providing said element, with a grip portion directly integrally obtained on the main body 2.

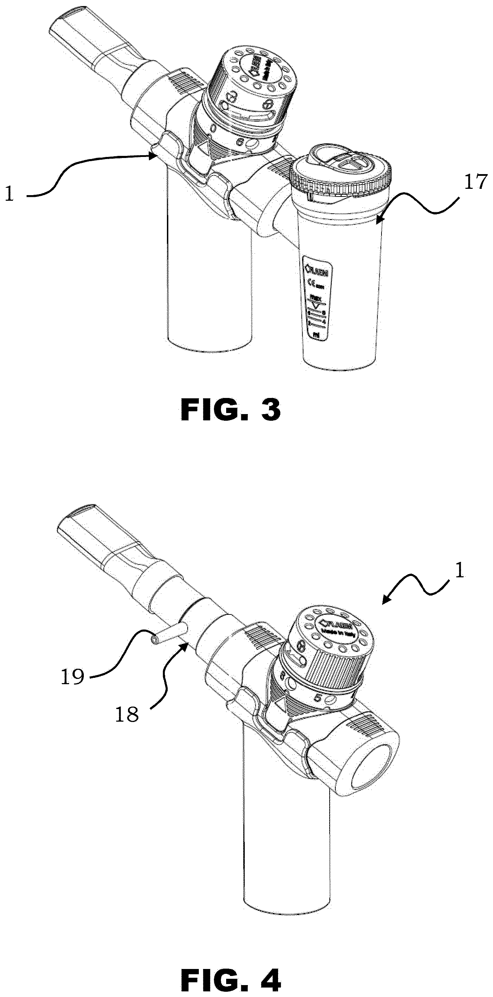

As visible in Figure 3, the device 1 may further be coupled with a nebulization cup 17, coupled at the opening 5 of the inlet plug 3, useful for humidifying secretions and therefore for improving the freedom of the airways.

Still furthermore, as visible in Figure 4, the device 1 may comprise a further tubular element 18 interposed between the hollow cylindrical portion 7 and the mouthpiece 8, and comprising a T connecting element 19, namely an outlet hollow side projection, generally adapted to be connected to a gauge, to allow the user, or preferably a third party such as a physiotherapist, to set the device 1.

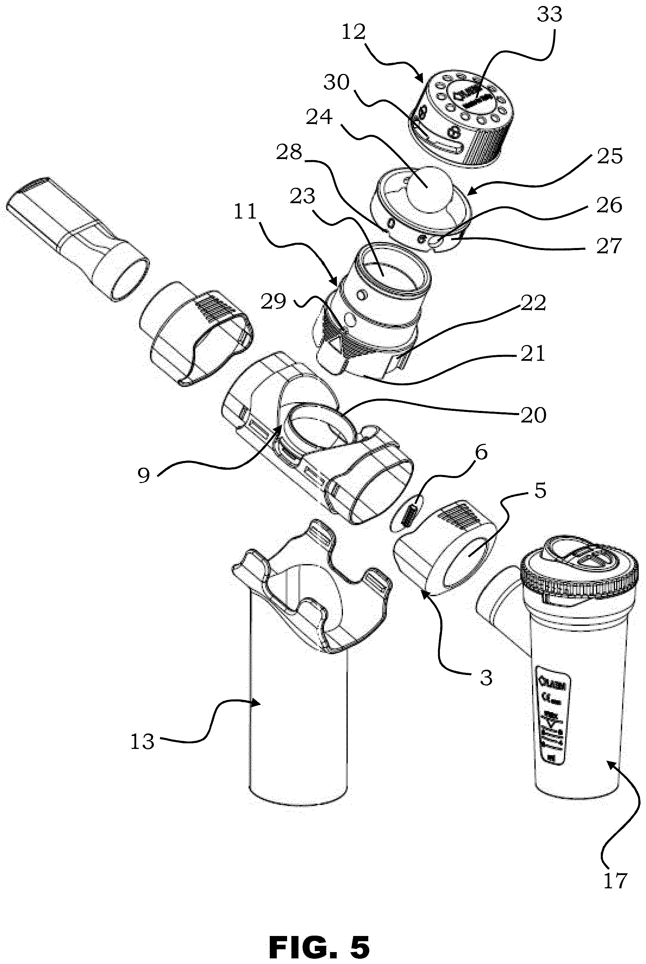

As visible in a more detailed manner in exploded Figure 5, the containment housing 9 is substantially boat-shaped, with a cylindrical coupling object 20 at the centre, whereon to shape couple the PEP element 11.

Specifically, the PEP element 11 is chimney-shaped, the base 21 of the PEP element 11 being adapted to be shape-coupled on the cylindrical coupling object 20.

Furthermore, at the base 21 a specific regulation recess 22 to correctly orient the fixing in the containment housing 9 and to facilitate correct assembly between the parts is provided.

When the orientation is correct, in a position opposite the register recess 22, the outlet plug 4 whereon the mouthpiece 8 is fitted abuts.

The PEP element 11 also comprises a truncated-conical, or a substantially vase-shaped housing seat 23, opposite the base 21, which develops toward the inside of the PEP element 11.

Oscillating means 24 are housed in the housing seat 23.

The oscillating means 24, in the present exemplifying and non-limiting embodiment, are represented by a spherical body 24, preferably metallic.

Nothing prevents from providing different oscillating bodies, for instance plate-shaped, or oscillating means comprising both a spherical body 24 and other elements, or other oscillating means made of different materials, also based on research developments.

The device 1 further comprises a setting ring nut 25 whereon calibrated holes 26 of different diameter are obtained, said calibrated holes 26 being adapted to allow performing a PEP therapy suitable for the patient.

In the present exemplifying embodiment five calibrated holes 26 are provided, generally numbered to allow a quick view thereof, but nothing prevents from providing a different number of calibrated holes 26.

A non-perforated area 27 is further provided to hermetically close the calibrated holes 26.

The setting ring nut 25 is coupled on an outer wall of the chimney-shaped PEP element 11 by conical interference.

The setting ring nut 25 also has a plurality of recesses 28 in the lower part which fit on a register pin 29 on the outer wall of the PEP element 11 to allow blocking the rotation thereof.

The OPEP element 12 is on top of the PEP element 11 at the housing seat 23 where the spherical body 24 is housed.

Specifically, the OPEP element 12, in the present embodiment, is coupled to the PEP element 11 by a cam 30 on the OPEP element 12 in which a position pin 35 of the PEP element 11 fits, said screwing taking place, as said, at the housing seat 23.

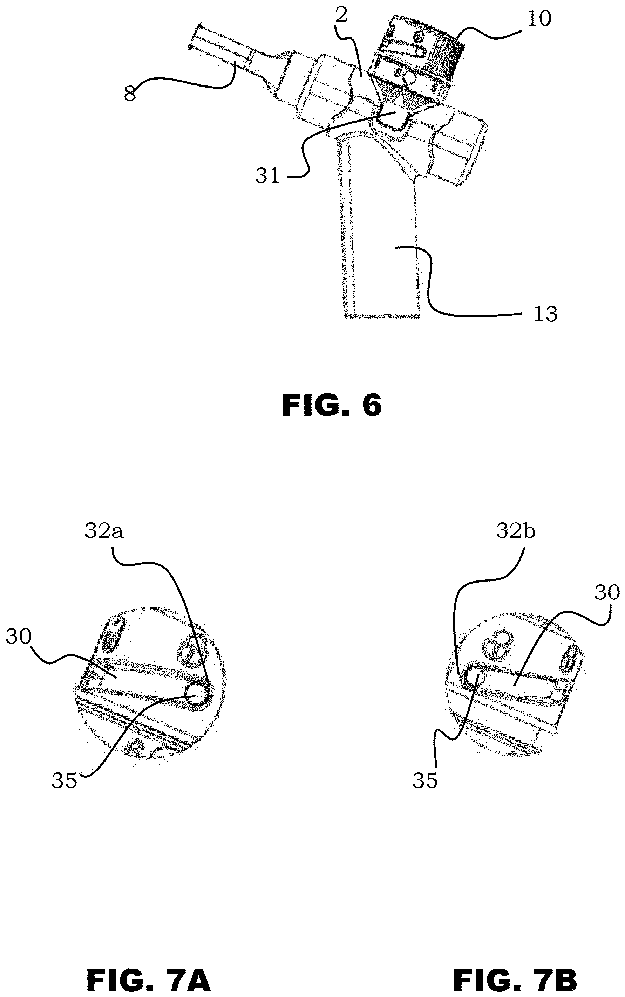

Figure 6 shows a side view of the device 1 in an assembled configuration, in which it is also possible to specifically appreciate an arrow-shaped projection 31 on the outer wall of the PEP element 11 and fitted in a respective notch of the containment housing 9, adapted to indicate one of the numbers on the setting ring nut 25, so as to a have visual confirmation of its correct positioning.

Furthermore, two positioning stops 32a and 32b, highlighted in Figures 7A and 7B, are provided, which define the cam 30 and between which the position pin 35 moves.

By means of the rotation, clockwise and counterclockwise respectively, of the OPEP element 12, this may advance approaching or moving away from the PEP element 11, and specifically of the spherical body 24, moving between a first position and a second position in a range defined by the positioning stops 32a and 32b.

In other words, the positioning stops 32a and 32b act as a tactile confirmation for the user that said first position and second positions have been reached.

In the first position, when the OPEP element 12 is closer to the spherical body 24, the top 33 of the OPEP element 12 abuts onto the spherical body 24, forcing it to remain fixed in housing seat 23.

Vice versa, in the second position, when the OPEP element 12 furthest from the spherical body 24, the top 33 of the OPEP element 12 and the spherical body 24 are spaced apart from each other, allowing the latter to move with respect to the housing seat 23.

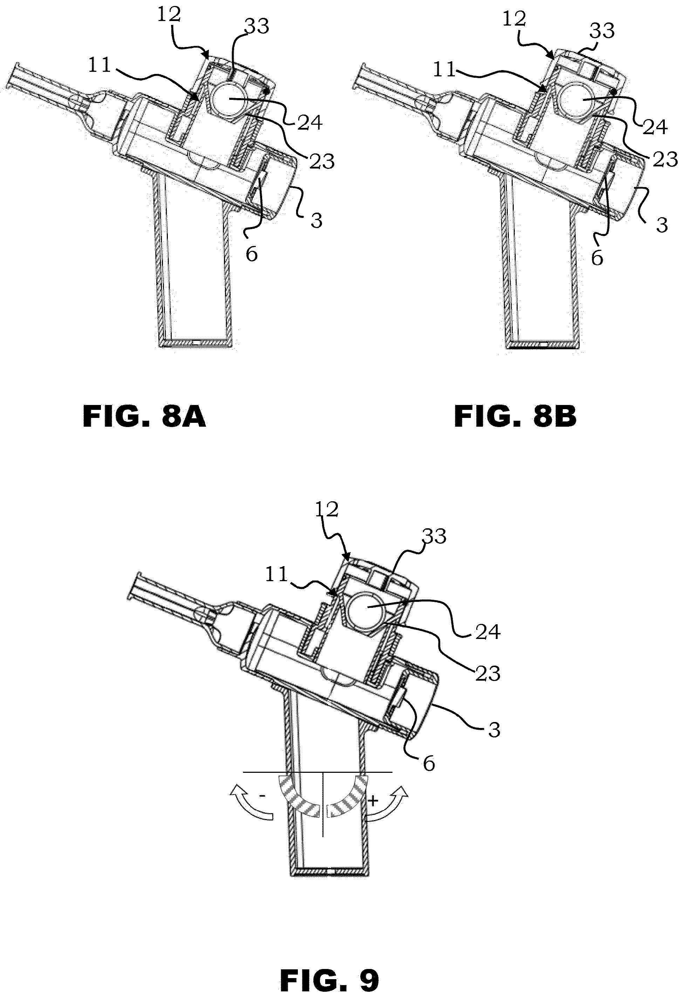

The operation of the device 1 will be now illustrated, with particular reference to the sectional views of Figures 8A and 8B.

Figure 8A shows a configuration in which the OPEP element 12 is in the first position and thus the top 33 thereof blocks the spherical body 24 in the truncated-conical housing seat 23.

In this case the air passage between the PEP element 11 and the OPEP element 12 is blocked.

The setting ring nut 25 is positioned to open one of the available calibrated holes 26.

During the patient's expiratory phase, the valve 6 in the inlet plug 3 closes, and the air is forced through the calibrated hole 26 selected by the setting ring nut 25.

In this configuration there is a classic PEP therapy.

Furthermore, in this configuration the device 1 can also be used, in the configuration shown in Figure 4 comprising the T connecting element 19.

As said, in this configuration generally a physiotherapist with a gauge connected to the T connecting element 19 through a connection tube chooses the most suitable calibrated hole 26 for the treatment to be administered to the patient.

Figure 8B shows a configuration in which the OPEP element 12 is in the second position that is obtained by counterclockwise rotating the OPEP element 12 between the two positioning stops 32a, 32b, and thus its top 33 is spaced apart from the spherical body 24, thus allowing the latter to move with respect to the housing seat 23.

In this second position, the spherical body 24 is set into vibration by the patient's expiratory act.

In this configuration, moreover, the setting ring nut 25 is positioned so as to correspond to the non-perforated area 27 to hermetically close the calibrated holes 26.

In this configuration there is an OPEP therapy.

During the patient's expiratory phase, the valve member 6 in the inlet plug 3 closes, and the pressurized air determines the fluctuation of the spherical body 24.

In both configurations, as seen, the inlet plug 3 and the valve member 6 allow, as said, the inlet of air into the device 1 and hermetically block the outgoing air, to put the device 1 under pressure both during the delivery of the PEP therapy and during the delivery of the OPEP therapy.

When the OPEP element 12 is in the second position, and therefore the chosen therapy is the OPEP one, the pressure necessary to make the spherical body 24 bounce is influenced by the position of the device 1, as explained hereinafter with reference to Figure 9.

If the device is gripped by holding the knob 13 in a vertical position with respect to the development of the cylindrical portion 14, the force applied by the respiratory act is the nominal one.

By tilting the device toward the outlet plug 4, in the area marked by the sign "-" in Figure 9, and thus by increasing the initial tilting angle, the force requested to move the spherical body 24 and the oscillation frequency are reduced.

Vice versa, by tilting device toward the inlet plug 3, in the area marked by the sign "+" in Figure 9, and thus by reducing the initial tilting angle, the force requested to move the spherical body 24 and the oscillation frequency are increased.

The greatest effort is therefore obtained when the spherical body 24 moves in a perfect vertical position with respect to the ground.

Advantageously, the present invention allows performing both a PEP therapy and an OPEP therapy with the same device.

Furthermore, advantageously a unique device is provided, thus reducing the overall production with significant savings in materials and production costs.

Furthermore, advantageously a device is provided that is intuitive to use for a user, whether specialized or not.

Still advantageously, the device according to the invention is substantially made of blocks that can be mutually fitted by using substantially standardized couplings.

This feature gives the device itself a high scalability, since it can also be coupled with other components such as, merely by way of example, a turbine module, which lights up multicolored LEDs both to encourage and strengthen the patients' respiratory system and to provide for differentiated starting according to the flux along a LED scale. In addition to the setting ring nut 25, it is also possible to provide a flow meter with a useful indicator for both physiotherapist and patient.

It is further possible to provide modules equipped with valves that allow the air to be channeled in a different way, or to apply a Positive Inspiratory Pressure, or PIP, useful for instance in sports for muscle strengthening.

It will be clear for the person skilled in the art that changes and variants can be made to the device according to the present invention, all of them falling within the scope defined by the appended claims.

For instance, nothing prevents from providing different conformations of the couplings and of the various housings provided for the components adopted.

## Claims

1. Respiratory physiotherapy device (1) comprising:
- a main body (2);
- a valve member (6) adapted to adjust an inlet and outlet air flow from said main body (2) allowing air to enter the device (1) and hermetically blocks the outletting air;
- fluid connection means (8) for connection with the respiratory tract of a patient, said fluid connection means (8) being connected to said main body (2);
- a convertible Positive Expiratory Pressure module (10) comprising oscillating means (24), said module (10) being connected to said main body (2) and switchable between a first position in which said oscillating means (24) are blocked in said module (10) for a PEP therapy and a second position in which said oscillating means (24) are movable in said module (10) for an OPEP therapy; and
- a setting ring nut (25) configured to be coupled to said module (10), said setting ring nut (25) comprising at least one calibrated hole (26).

2. Device (1) according to claim 1, wherein said module (10) comprises:
- a first element (11) coupled to said main body (2), said first element (11) comprising a housing seat (23) for said oscillating means (24), and
- a second element (12) coupled to the first element (11) and adapted to constrain and release said oscillating means (24) from said housing seat (23).

3. Device (1) according to claim 1 or 2, wherein said second element (12) comprises at least one cap portion adapted to be on top of said oscillating means (24) on said housing seat (23) of said first element (11).

4. Device (1) according to any one of claims 1 to 3, wherein said oscillating means (24) comprise a spherical body, preferably metallic.

5. Device (1) according to any one of claims 1 to 4, wherein said setting ring nut (25) configured to be coupled to said module (10) comprises calibrated holes (26) of different diameter.

6. Device (1) according to any one of claims 1 to 5, further comprising an inlet plug (3) coupled to said main body (2) at said valve member (6).

7. Device (1) according to any one of claims 1 to 6, further comprising an outlet plug (4) coupled to said main body (2) at said fluid connection means (8).

8. Device (1) according to any one of claims 1 to 7, further comprising at least one T connecting element (19) coupled between said main body (2) and said fluid connection means (8), said T connecting element (19) being adapted to be connected to a gauge.

9. Device (1) according to any one of claims 1 to 8, wherein said main body (2) further comprises a containment housing (9) for said module (10).

10. Device (1) according to any one of claims 1 to 9, further comprising at least one grip knob (13) adapted to be coupled to said main body (2).

## Patentansprüche

1. Physiotherapeutisches Atemgerät (1), das umfasst:
- einen Hauptkörper (2);
- ein Ventilelement (6), das dazu ausgelegt ist, einen Einlass- und Auslassluftluftstrom aus dem Hauptkörper (2) anzupassen, um Luft den Eintritt in das Gerät (1) zu ermöglichen, und das die Auslassluft hermetisch blockiert;
- ein Fluidverbindungsmittel (8) zur Verbindung mit den Atemwegen eines Patienten, wobei das Fluidverbindungsmittel (8) mit dem Hauptkörper (2) verbunden ist;
- ein umkehrbares Modul (10) für positiven Ausatmungsdruck, das ein Oszillationsmittel (24) umfasst, wobei das Modul (10) mit dem Hauptkörper (2) verbunden und zwischen einer ersten Stellung, in der das Oszillationsmittel (24) in dem Modul (10) für eine PEP-Therapie blockiert ist, und einer zweiten Stellung schaltbar ist, in der das Oszillationsmittel (24) in dem Modul (10) für eine OPEP-Therapie beweglich ist; und
- eine Einstellringmutter (25), die dazu ausgestaltet ist, mit dem Modul (10) verbunden zu werden, wobei die Einstellringmutter (25) zumindest ein kalibriertes Loch (26) umfasst.

2. Gerät (1) nach Anspruch 1, wobei das Modul (10) umfasst:
- ein erstes Element (11), das mit dem Hauptkörper (2) verbunden ist, wobei das erste Element (11) eine Gehäuseaufnahme (23) für das Oszillationsmittel (24) umfasst, und
- ein zweites Element (12), das mit dem ersten Element (11) verbunden und dazu ausgelegt ist, das Oszillationsmittel (24) zu begrenzen und aus der Gehäuseaufnahme (23) und freizugeben.

3. Gerät (1) nach Anspruch 1 oder 2, wobei das zweite Element (12) zumindest einen Kappenabschnitt umfasst, der dazu ausgelegt ist, oben auf dem Oszillationsmittel (24) auf der Gehäuseaufnahme (23) des ersten Elements (11) platziert zu sein.

4. Gerät (1) nach einem der Ansprüche 1 bis 3, wobei das Oszillationsmittel (24) einen kugelförmigen Körper umfasst, der vorzugsweise metallisch ist.

5. Gerät (1) nach einem der Ansprüche 1 bis 4, wobei die Einstellringmutter (25), die dazu ausgestaltet ist, mit dem Modul (10) verbunden zu werden, kalibrierte Löcher (26) mit unterschiedlichen Durchmessern umfasst.

6. Gerät (1) nach einem der Ansprüche 1 bis 5, das ferner einen Einlassstopfen (3) umfasst, der an dem Ventilelement (6) mit dem Hauptkörper (2) verbunden ist.

7. Gerät (1) nach einem der Ansprüche 1 bis 6, das ferner einen Auslassstopfen (4) umfasst, der an dem Fluidverbindungsmittel (8) mit dem Hauptkörper (2) verbunden ist.

8. Gerät (1) nach einem der Ansprüche 1 bis 7, das ferner zumindest ein T-Verbindungselement (19) umfasst, das zwischen dem Hauptkörper (2) und dem Fluidverbindungsmittel (8) verbunden ist, wobei das T-Verbindungsmittel (19) dazu ausgelegt ist, mit einer Messvorrichtung verbunden zu werden.

9. Gerät (1) nach einem der Ansprüche 1 bis 8, wobei der Hauptkörper (2) ferner ein Begrenzungsgehäuse (9) für das Modul (10) umfasst.

10. Gerät (1) nach einem der Ansprüche 1 bis 9, das ferner zumindest einen Greifknopf (13) umfasst, der dazu ausgelegt ist, mit dem Hauptkörper (2) verbunden zu werden.

## Revendications

1. Dispositif de physiothérapie respiratoire (1) comprenant :
- un corps principal (2) ;
- un organe de vanne (6) adapté pour régler un flux d'air d'entrée et de sortie dudit corps principal (2) permettant à l'air d'entrer dans le dispositif (1) et bloque hermétiquement l'air sortant ;
- des moyens de liaison fluidique (8) pour la liaison avec les voies respiratoires d'un patient, lesdits moyens de liaison fluidique (8) étant reliés audit corps principal (2) ;
- un module (10) de pression expiratoire positive convertible comprenant des moyens oscillants (24), ledit module (10) étant relié audit corps principal (2) et pouvant basculer entre une première position dans laquelle lesdits moyens oscillants (24) sont bloqués dans ledit module (10) pour une thérapie PEP et une deuxième position dans laquelle lesdits moyens oscillants (24) sont mobiles dans ledit module (10) pour une thérapie OPEP ; et
- un écrou à anneau de réglage (25) configuré pour être couplé audit module (10), ledit écrou à anneau de réglage (25) comprenant au moins un trou calibré (26).

2. Dispositif (1) selon la revendication 1, dans lequel ledit module (10) comprend :
- un premier élément (11) couplé audit corps principal (2), ledit premier élément (11) comprenant un siège de logement (23) pour lesdits moyens oscillants (24), et
- un deuxième élément (12) couplé au premier élément (11) et adapté pour contraindre et libérer lesdits moyens oscillants (24) dudit siège de logement (23).

3. Dispositif (1) selon la revendication 1 ou 2, dans lequel ledit deuxième élément (12) comprend au moins une partie de capuchon adaptée pour être au-dessus desdits moyens oscillants (24) sur ledit siège de logement (23) dudit premier élément (11).

4. Dispositif (1) selon l'une quelconque des revendications 1 à 3, dans lequel lesdits moyens oscillants (24) comprennent un corps sphérique, de préférence métallique.

5. Dispositif (1) selon l'une quelconque des revendications 1 à 4, dans lequel ledit écrou à anneau de réglage (25) configuré pour être couplé audit module (10) comprend des trous calibrés (26) de diamètre différent.

6. Dispositif (1) selon l'une quelconque des revendications 1 à 5, comprenant en outre un bouchon d'entrée (3) couplé audit corps principal (2) au niveau dudit organe de vanne (6).

7. Dispositif (1) selon l'une quelconque des revendications 1 à 6, comprenant en outre un bouchon de sortie (4) couplé audit corps principal (2) au niveau desdits moyens de liaison fluidique (8).

8. Dispositif (1) selon l'une quelconque des revendications 1 à 7, comprenant en outre au moins un élément de liaison en T (19) couplé entre ledit corps principal (2) et lesdits moyens de liaison fluidique (8), ledit élément de liaison en T (19) étant adapté pour être relié à une jauge.

9. Dispositif (1) selon l'une quelconque des revendications 1 à 8, dans lequel ledit corps principal (2) comprend en outre un boîtier de confinement (9) pour ledit module (10).

10. Dispositif (1) selon l'une quelconque des revendications 1 à 9, comprenant en outre au moins un bouton de préhension (13) adapté pour être couplé audit corps principal (2).
